# EUROPÄISCHE PATENTSCHRIFT

(11) **EP 3 265 038 B1**
(45) Veröffentlichungstag und Bekanntmachung des Hinweises auf die Patenterteilung: **01.05.2024**
(21) Anmeldenummer: 16710691.3
(22) Anmeldetag: 04.03.2016
(51) Int. Cl.: A61F 2/966, A61F 2/90, A61F 2/82

(54) **IMPLANTATEINFÜHRSYSTEM**
IMPLANT INSERTION SYSTEM
SYSTÈME D'INTRODUCTION D'UN IMPLANT

(30) Priorität: 05.03.2015 DE 102015103240
(43) Veröffentlichungstag der Anmeldung: 10.01.2018
(73) Patentinhaber: Phenox GmbH, 44801 Bochum (DE)
(72) Erfinder: HENKES, Hans, 70192 Stuttgart (DE); MONSTADT, Hermann, 44797 Bochum (DE); HANNES, Ralf, 44137 Dortmund (DE); ROLLA, Stefan, 44869 Bochum (DE); SALIN, Manuel, 44801 Bochum (DE)
(74) Vertreter: Schöneborn, Holger
(86) Internationale Anmeldenummer: PCT/EP2016/054692
(87) Internationale Veröffentlichungsnummer: WO 2016/139357

(56) Entgegenhaltungen:
- EP-A2- 2 628 470
- WO-A1-2012/009006
- US-A1- 2006 155 357
- US-A1- 2012 226 343
- US-A1- 2014 277 361

## Beschreibung

Die Erfindung betrifft eine Vorrichtung zur Einbringung eines Implantates in Blutgefäße oder Hohlorgane des menschlichen oder tierischen Körpers, umfassend ein Implantat und einen Ablöseschlauch, wobei das Implantat in der Weise verformbar ist, dass es in einem Mikrokatheter eine Form mit vermindertem Durchmesser annimmt und am Ort der Implantation nach Wegfall des äußeren Zwangs durch den Mikrokatheter unter Anpassung an den Durchmesser des Blutgefäßes oder Hohlorganes expandiert, und der Ablöseschlauch ein in Längsrichtung der Vorrichtung verlaufendes Lumen aufweist, durch welches ein Einführdraht längsverschieblich führbar ist.

Arteriovenöse Fehlbildungen können in einem Patienten zu erheblichen Beeinträchtigungen und Gefährdungen bis hin zum Tode führen. Dies gilt insbesondere für arteriovenöse Fisteln und Aneurysmen, besonders dann, wenn sie im zerebralen Bereich auftreten. In der Regel versucht man, solche Fehlbildungen durch Implantate zu verschließen. Derartige Implantate werden zumeist auf endovaskulärem Weg mit Hilfe von Kathetern gesetzt.

Insbesondere bei Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma letztlich verschließt. Diese Behandlungsmethode ist allerdings nur für Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden und Schäden in anderen Bereichen des Gefäßsystems herbeizuführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, haben aber eine Reihe von Nachteilen.

Zum einen ist dies die weitmaschige Struktur, die den Blutzutritt in das Aneurysma unbeeinträchtigt zulässt. Ist das Aneurysma aber nicht hinreichend mit dem Okklusionsmittel ausgefüllt, bleibt der Druck auf die Gefäßwandung unvermindert bestehen. Eine Nachbehandlung ist unter diesen Umständen nur schwer möglich, da der Stent den Zugang zum Aneurysma beeinträchtigt und die Einbringung weiterer Okklusionsmittel behindert.

Ein weiterer Nachteil ist die fehlende Anpassbarkeit des Stents an seinen Einsatzort. Für eine optimale Funktion sollte sich der Stent dicht an die Gefäßwandung anlegen, ohne jedoch einen übermäßigen Druck auf die Wandung auszuüben. Im Gegensatz zu Stents, die eine Aufweitung des Gefäßes bei Stenosen bewirken sollen, sind diese Stents eher als eine Art Manschette zu verstehen, die das Gefäßlumen und die Endothelwand des Gefäßes möglichst wenig beeinflussen soll.

Aus Drahtgeflecht bestehende Stents sind insbesondere für den Einsatz im koronaren Bereich seit langem bekannt. Diese Stents werden in der Regel als Rundgeflecht gefertigt, wobei die einzelnen Drahtfilamente in gegenläufigen spiralförmigen bzw. helixförmigen Lagen die Stentwandung ausbilden. Es entsteht ein Maschengeflecht, das in radialer Richtung sowohl abstützt als auch für Blut durchlässig ist.

Derartige als Rundgeflecht aus Filamenten bestehende Stents werden beim Einsatz zur Behandlung von Stenosen häufig mit Hilfe eines Ballons am Einsatzort hydraulisch aufgeweitet und an der Gefäßwandung fixiert. Während der Einbringung dient der an einem Einführdraht befestigte Ballon als Transportvehikel, auf das der Stent aufgecrimpt ist. Für Implantate, die zur Beeinflussung bzw. Kanalisierung des Blutflusses im zerebralen Bereich dienen, ist aber ein Implantat von Vorteil, das sich selbständig an den Gefäßdurchmesser anpasst und an die Gefäßwandung anlegt.

In der WO 2008/107172 A1 wird ein Implantat beschrieben, bei dem das Geflecht in einem Mikrokatheter eine gelängte Form mit vermindertem Durchmesser aufweist und am Implantationsort unter Anpassung an den Gefäßdurchmesser und Zunahme der Geflechtdichte expandiert, wobei die an den Implantatenden herausstehenden Filamentenden zumindest paarweise zusammengeführt und miteinander verbunden sind. Auf diese Weise wurde ein Implantat zur Verfügung gestellt, das in der Lage ist, sich an den jeweiligen Gefäßdurchmesser anzupassen, wobei die Filamentenden atraumatisch sind.

Gemäß diesem Stand der Technik sind an den zusammengeführten Filamentenden Verbindungselemente angebracht, die mit Halteelementen nach dem Schlüssel-Schloss-Prinzip zusammenwirken. Das Halteelement, über welches das Implantat mit einem Einführdraht gekoppelt ist, weist Ausnehmungen auf, in die die Verbindungselemente eingepasst sind. Die Verbindungselemente weisen eine Verdickung, beispielsweise eine Kugelform auf, sodass sie in den Ausnehmungen des Halteelements formschlüssig gehalten werden. Die Fixierung in den Ausnehmungen kann mit Hilfe eines Schlauchs erfolgen, der formschlüssig über das Halteelement mit den eingepassten Verbindungselementen gezogen ist. Dieser Schlauch wird nach Erreichen der Endposition des Implantates in Richtung proximal zurückgezogen, und das Implantat wird abgelöst. Anschließend können Einführdraht mit Halteelement, Schlauch und Katheter zurückgezogen und aus dem Körper entfernt werden.

Der beschriebene Stand der Technik hat sich grundsätzlich bewährt, in Einzelfällen kann es jedoch vorkommen, dass sich nicht sämtliche Verbindungselemente nach Zurückziehen des Schlauches aus den für sie vorgesehenen Ausnehmungen des Halteelementes lösen, beispielsweise weil eine Verkantung auftritt, durch die ein Verbindungselement weiterhin in der Ausnehmung des Halteelementes gehalten wird. In einem solchen Fall öffnet sich das Implantat an seinem proximalen Ende nicht so rasch, wie es wünschenswert wäre, und löst sich vom Halteelement unter Umständen erst nach weiterer Bewegung des Einführdrahtes. Wünschenswert ist hingegen ein Ablösesystem, bei dem sich das Implantat unmittelbar nach Zurückziehen des Schlauchs vollständig vom Halteelement löst und dadurch freigesetzt wird. Es stellt sich somit die Aufgabe, ausgehend von dem in der WO 2008/107172 A1 beschriebenen Stand der Technik das Ablösesystem weiter zu optimieren.

Im Rahmen der Beschreibung wird unter dem Begriff proximal dem behandelnden Arzt zugewandt verstanden, d. h. das proximale Ende weist in Richtung Körperäußeres. Umgekehrt bedeutet distal dem Arzt abgewandt, das distale Ende liegt also in Richtung Körperinneres.

Das Implantat selbst ist typischerweise ein Geflecht aus einer Vielzahl an Geflechtdrähten, die spiral- oder helixförmig verlaufen, wobei sich gegenläufig verlaufende Geflechtdrähte gegenseitig schneiden und ein Maschengeflecht ausbilden. Derartige Geflechtstrukturen sind aus dem Stand der Technik hinreichend bekannt, beispielsweise aus der oben bereits zitierten WO 2008/107172 A. US 2014/277361 A1 zeigt Systeme und Anwendungsmethoden für Stents. Die Systeme können einen Katheter, ein Kernelement und ein Eingriffselement sowie einen Stent umfassen. Der Katheter kann eine Innenwand haben. Das Kernelement kann sich innerhalb des Katheters erstrecken und ein distales Segment aufweisen. Das Eingriffselement kann am distalen Segment befestigt werden. Darüber hinaus kann der Stent innerhalb des Katheters positioniert werden und sich über das Eingriffselement erstrecken. Der Stent kann eine axiale Länge haben, die größer ist als eine axiale Länge des Eingriffselements. Das Eingriffselement kann einen expandierten Zustand aufweisen, in dem das Eingriffselement den Stent gegen die Innenwand des Katheters drückt, um mit dem Stent in Eingriff zu kommen und den Stent zusammen mit dem Kernelement relativ zum Katheter zu bewegen.

Erfindungsgemäß wird ein vereinfachtes Ablösesystem zur Verfügung gestellt, das auf Reibschluss (Kraftschluss) beruht. Es handelt sich um eine Vorrichtung zur Einbringung eines Implantates in Blutgefäße oder Hohlorgane des menschlichen oder tierischen Körpers, umfassend ein Implantat und einen Ablöseschlauch, wobei das Implantat in der Weise verformbar ist, dass es in einem Mikrokatheter eine Form mit vermindertem Durchmesser annimmt und am Ort der Implantation nach Wegfall des äußeren Zwangs durch den Mikrokatheter unter Anpassung an den Durchmesser des Blutgefäßes oder Hohlorganes expandiert, und der Ablöseschlauch ein in Längsrichtung der Vorrichtung verlaufendes Lumen aufweist, durch welches ein Einführdraht längsverschieblich führbar ist, wobei der Ablöseschlauch in das proximale Ende des Implantates hineinragt und eine elastische Kontaktfläche zwischen der Innenseite des Implantates und der Außenseite des Ablöseschlauches vorliegt, sodass ein Reibschluss zwischen Implantat und Ablöseschlauch erzeugt wird, der eine Längsverschieblichkeit des Implantates innerhalb des Mikrokatheters durch Längsbewegung des Ablöseschlauches nach distal oder proximal herbeiführt.

Der Ablöseschlauch umschließt nicht das proximale Ende des Implantates bzw. vom Implantat ausgehende Haltedrähte, sondern ragt in dieses hinein. Dabei wird eine reibschlüssige Verbindung zwischen Ablöseschlauch und Implantat hergestellt, sodass es möglich ist, durch Vorschieben oder Zurückziehen des Ablöseschlauches das Implantat in gleicher Weise nach distal oder proximal zu bewegen. Der Reibschluss zwischen Ablöseschlauch und Implantat wird dadurch herbeigeführt, dass der in das Implantat hineinragende Teil des Ablöseschlauches zumindest teilweise eine elastische Kontaktfläche aufweist. Bevorzugt handelt es sich dabei um eine Zwischenlage, die im in das Implantat hineinragenden Teil des Ablöseschlauches auf der Außenseite des Ablöseschlauches vorhanden ist und auch als Pad bezeichnet werden kann. Die elastische Kontaktfläche, bevorzugt die Zwischenlage, sorgt für eine reibschlüssige Verbindung mit dem Implantat. Eine solche Ausführungsform ist besonders einfach aufgebaut und bedarf keiner Anformung von zusätzlichen Elementen, die einen Formschluss herbeiführen.

Die Freisetzung des Implantates erfolgt dadurch, dass das Implantat aus dem Mikrokatheter herausgeschoben bzw. der Mikrokatheter relativ zum Implantat nach proximal zurückgezogen wird. Da das Implantat eine natürliche Tendenz hat, sich nach Wegfall des äußeren Zwangs durch den Mikrokatheter radial aufzuweiten, löst sich das Implantat nach Freisetzung aus dem Mikrokatheter vom elastischen Pad und legt sich an die Innenwand des Gefäßes oder Hohlorgans an. Der Ablöseschlauch, der Mikrokatheter sowie der ggf. durch den Ablöseschlauch hindurch verlaufende Einführdraht können anschließend zurückgezogen und aus dem Körper entfernt werden.

Der Ablöseschlauch weist ein Innenlumen auf, durch das sich der Einführdraht erstrecken kann. Typischerweise wird, nachdem der Einführdraht an die gewünschte Position gebracht wurde, der Mikrokatheter über den Einführdraht bis an den Zielort geschoben. Anschließend kann der Ablöseschlauch samt reibschlüssig hiermit verbundenem Implantat in Richtung distal durch den Mikrokatheter hindurch vorgeschoben werden. Das Zurückziehen und Entfernen des Einführdrahtes ist sowohl vor, als auch während oder nach der Freisetzung des Implantates möglich. Wenn man verhindern will, dass sich die Einführdrahtspitze in distal liegende Gefäße bewegt, kann ein Zurückziehen des Einführdrahtes bereits vor oder während der Implantatfreisetzung erfolgen.

Die elastische Kontaktfläche/Zwischenlage verläuft typischerweise ringförmig bzw. zirkulär um den Ablöseschlauch. Mit anderen Worten ist der Ablöseschlauch in einem Bereich radial umlaufend von der elastischen Kontaktfläche/Zwischenlage umgeben, die für die reibschlüssige Verbindung mit dem Implantat sorgt. Beim Implantat handelt es sich typischerweise um ein Geflecht aus Geflechtdrähten, obgleich auch anders geartete Implantate wie etwa rohrförmige oder geschnittene Implantate nicht ausgeschlossen sind.

Zur Herstellung der reibschlüssigen Verbindung mit dem Implantat können ein oder mehrere Zwischenlagen (Pads) vorgesehen sein, wobei sich die Pads über eine gewisse Länge innerhalb des Implantates erstrecken können. In der Regel wird die Zahl der Pads 1 oder 2 betragen, wobei im Falle der Verwendung von zwei Pads diese entsprechend kürzer ausgebildet sein können. Dort, wo die Zwischenlagen auf dem Ablöseschlauch angebracht sind, weist dieser einen größeren Außendurchmesser auf als in den angrenzenden Bereichen ohne Zwischenlage.

Das für die Zwischenlage/das Pad verwendete Material muss elastisch sein, um ausreichend hohe Reibungskräfte zwischen Ablöseschlauch und Implantat herbeizuführen, die ein Vorschieben oder Zurückziehen des Implantates über den Ablöseschlauch ermöglichen, ohne dass sich das Implantat relativ zum Ablöseschlauch in Längsrichtung bewegt oder gar vom Ablöseschlauch löst. Als Materialien für die elastische Zwischenlage kommen viele verschiedene Materialien in Frage, insbesondere kann es sich um ein Elastomer handeln. Verwendbar sind beispielsweise Gummi, Kautschuk oder Silikon.

Die Zwischenlage kann auch aus einem Polymermaterial wie Polytetrafluorethylen, Polyester, Polyamiden, Polyurethanen oder Polyolefinen hergestellt sein. Besonders bevorzugt sind Polycarbonaturethane. Dabei wird die Zwischenlage vorzugsweise durch Elektrospinnen erzeugt. Hierbei werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3000 nm. Durch Elektrospinnen gewonnene Schichten sind sehr gleichmäßig ausgebildet, zäh und mechanisch belastbar. Im Zusammenhang mit der Schaffung einer Zwischenlage mittels Elektrospinnings wird insbesondere auf die WO 2008/049386, die DE 28 06 030 A1 und die darin genannte Literatur hingewiesen.

Ebenso ist es möglich, die Zwischenlage aus demselben Material zu fertigen wie den Ablöseschlauch, der bevorzugt aus einem Polyimid besteht. In diesem Fall kann die Zwischenlage einstückig mit dem Ablöseschlauch ausgebildet sein, wobei der Ablöseschlauch im Bereich der Zwischenlage einen entsprechend größeren Außendurchmesser aufweist als in angrenzenden Bereichen des Ablöseschlauchs ohne Zwischenlage. Erfindungsgemäß ist auch eine einstückig mit dem Ablöseschlauch ausgebildete Zwischenlage von dem Begriff Zwischenlage erfasst, sofern die Zwischenlage elastisch ist und einen ausreichend hohen Reibschluss mit dem Implantat erzeugt. Möglich ist auch, dass der Ablöseschlauch sich in dem in das Implantat erstreckenden Abschnitt einen einheitlichen Querschnitt aufweist, solange ein ausreichend starker Reibschluss zwischen Implantat und Ablöseschlauch erzeugt wird. Beispielsweise kann auch ein distaler Abschnitt des Ablöseschlauches einen verringerten Durchmesser aufweisen, sodass dieser distale Abschnitt in Längsrichtung in das Implantat eingeführt werden kann, um hier die gewünschte reibschlüssige Verbindung herbeizuführen.

Zwischen der elastischen Zwischenlage und dem eigentlichen Ablöseschlauch kann zusätzlich röntgensichtbares Material vorgesehen sein, um die Visualisierung des Implantateinbringungsvorgangs zu verbessern. Besonders bevorzugt ist eine Wendel, auch Coil genannt, aus röntgensichtbarem Material, da dieses ausreichend biegeweich ist, um einen problemlosen Vorschub des Implantats zu gewährleisten. Möglich ist aber auch röntgensichtbares Material in anderer Form, beispielsweise in Form einer auf den Ablöseschlauch aufgesteckten Hülse. Als röntgensichtbares Material sind Platin und Platinlegierungen bevorzugt. Insbesondere kann die elastische Zwischenlage mit einer Polymerschicht versehen werden, bevorzugt aus Polycarbonaturethan, besonders bevorzugt erzeugt mittels Elektrospinnings, wie vorstehend beschrieben.

Gemäß einer weiteren Variante wird ein Ablösedraht in dem Bereich, in dem der Ablöseschlauch in das proximale Ende des Implantats hineinragt und in dem eine elastische Kontaktfläche zwischen der Innenseite des Implantates und der Außenseite des Ablöseschlauchs vorliegt, um das Implantat geschlungen, sodass der Ablösedraht eine Verstärkung der Reibungskräfte zwischen Implantat und Ablöseschlauch erzeugt, wobei der Ablösedraht elektrolytisch korrodierbar ausgebildet ist.

Gemäß dieser Variante wird das Implantat nicht oder nicht ausschließlich durch den Mikrokatheter in einer komprimierten Form gehalten, sondern (auch) durch einen Ablösedraht, der um das Implantat sowie den in das Implantat eingeführten Ablöseschlauch mit elastischer Kontaktfläche geschlungen ist. Dabei wird der Ablösedraht auf dem Implantat quasi festgeschnürt. Solange der Ablösedraht nicht gelöst ist, kann somit keine endgültige Freisetzung des Implantats erfolgen, weil die Reibungskräfte zwischen Implantat und Ablöseschlauch zu hoch sind. Der Ablöseschlauch weist zur Herbeiführung hoher Reibungskräfte elastische Kontaktflächen, bevorzugt elastische Zwischenlagen/Pads wie oben beschrieben auf. Anders als bei der zuvor beschriebenen Variante wird aber das Implantat auch dann noch reibschlüssig auf dem Ablöseschlauch gehalten, wenn der Mikrokatheter bereits vom Implantat zurückgezogen bzw. das Implantat aus dem Mikrokatheter herausgeschoben wurde. Auf diese Weise wird eine besonders hohe Sicherheit bei der Platzierung des Implantats gewährleistet, da auch nach Freisetzung des Implantats aus dem Mikrokatheter das Implantat zunächst noch in seiner komprimierten Form verbleibt und ein Zurückziehen des Implantats in den Mikrokatheter daher weiterhin möglich ist.

Der Ablösedraht umgibt das Implantat in der axialen Position, in der sich die elastischen Kontaktflächen, bevorzugt die elastischen Zwischenlagen/Pads befinden. Der Ablösedraht kann unmittelbar dort das Implantat umschnüren, wo sich eine elastische Kontaktfläche/ein Pad befindet. Möglich ist auch die Umschnürung des Implantates in einer axialen Position des Implantates, die zwischen zwei elastischen Kontaktflächen/Pads liegt.

Der das Implantat umschlingende Ablösedraht ist an zumindest einer Ablösestelle elektrolytisch korrodierbar ausgebildet. Diese Ablösestelle sollte sich in dem Abschnitt des Ablösedrahtes befinden, der in Form einer Schlaufe/Schlinge um das Implantat herumgelegt ist. Anstelle einer elektrolytischen Korrodierbarkeit ist auch eine thermische Ablösung denkbar, bei der die Ablösestelle so weit erwärmt wird, dass der Ablösedraht durchtrennt wird. Die elektrolytische Korrosion oder auch die Erwärmung wird durch Anlegen an eine Spannungsquelle herbeigeführt. Hierzu werden bevorzugt die Enden des Ablösedrahtes nach proximal geführt, wo der Anschluss erfolgen kann. Mit anderen Worten verläuft der Ablösedraht von proximal bis zur Position des in das Implantat eingeführten Ablöseschlauchs, bildet dort eine Schlinge oder Schlaufe um das Implantat und verläuft wieder zurück in proximaler Richtung. Wenn eine Spannung an den Ablösedraht angelegt wird, erfolgt eine elektrolytische Korrosion des Ablösedrahtes an der Ablösestelle, sodass der Ablösedraht an dieser Position durchtrennt wird. Der Ablösedraht ist somit nicht mehr in der Lage, das Implantat auf den Ablöseschlauch zu pressen. Wegen der dem Implantat aufgeprägten Tendenz, nach Wegfall eines äußeren Zwangs zu expandieren, löst sich das Implantat vom Ablöseschlauch und wird damit endgültig freigesetzt. Mikrokatheter, Ablöseschlauch, Einführdraht und Ablösedraht können anschließend zurückgezogen werden.

Damit eine elektrolytische Korrosion des Ablösedrahtes herbeigeführt werden kann, kann es sinnvoll sein, den Ablösedraht an zumindest einer Position so zu schwächen, dass die elektrolytische Korrosion bevorzugt an dieser Ablösestelle erfolgt. Diese Ablösestelle kann z. B. einen kleineren Querschnitt aufweisen als andere Bereiche des Ablösedrahtes. Darüber hinaus ist es möglich, Teile des Ablösedrahtes aus einem Material zu fertigen, das sich bei Anlegen einer elektrischen Spannung besonders gut auflöst. Die für die Ablösestelle verwendbaren Materialien entsprechen denen, wie sie auch beispielsweise bei elektrolytisch ablösbaren Implantaten Verwendung finden. Hierzu gehören Stahl, Magnesium oder Magnesiumlegierungen sowie Cobalt-Chrom-Legierungen. Letztere werden in der WO 2011/147567 A1 beschrieben, auf die insoweit Bezug genommen wird. Um eine Konzentration des Stromflusses auf die Ablösestelle zu erreichen, kann der Ablösedraht außerhalb der Ablösestelle ganz oder teilweise durch eine Ummantelung isoliert sein.

Bei der beschriebenen Variante mit Ablösedraht wird zur Einbringung des Implantats so vorgegangen, dass das am Ablöseschlauch festgelegte Implantat durch den Mikrokatheter an den Zielort vorgeschoben wird, indem der Ablöseschlauch nach distal bewegt wird. Die reibschlüssige Verbindung zwischen Ablöseschlauch und Implantat sorgt dafür, dass sich das Implantat entsprechend dem Ablöseschlauch mitbewegt. Am Zielort wird der Mikrokatheter nach proximal zurückgezogen oder das Implantat nach distal aus dem Mikrokatheter herausgeschoben. Aufgrund der Umschnürung durch den Ablösedraht wird das Implantat weiterhin auf dem Ablöseschlauch gehalten, bis der behandelnde Arzt die endgültige Freisetzung des Implantats beschließt. Hierzu wird eine Spannung an den Ablösedraht angelegt, woraufhin dieser durchtrennt wird und das Implantat zur Expansion freigibt.

Gemäß einer bevorzugten Ausführungsform variiert der Außendurchmesser des Ablöseschlauchs zwischen proximalem und distalem Ende, wobei sich die Variation des Außendurchmessers auf die außerhalb des Implantates liegenden Bereiche des Ablöseschlauches bezieht. Diese zuletzt genannten Bereiche werden im Folgenden als distaler Abschnitt bezeichnet. Durch eine Variation des Außendurchmessers des Ablöseschlauchs zwischen proximalem und distalem Ende werden die Vorteile einer hohen Flexibilität und einer problemlosen, vorhersehbaren Ablösbarkeit miteinander kombiniert. In Teilabschnitten des Ablöseschlauchs, insbesondere in einem Bereich, der sich proximal an den distalen Abschnitt anschließt, welcher unmittelbar in das Implantat eingreift, ist eine hohe Flexibilität von besonderer Bedeutung, damit die Gesamtvorrichtung beim Einführen auch feinen Gefäßwindungen folgt. Aus diesem Grund ist hier ein kleiner Außendurchmesser sinnvoll. Auf der anderen Seite sollten die noch weiter proximal liegenden Abschnitte des Ablöseschlauchs eine ausreichende Widerstandskraft gegen eine unerwünschte Längung aufweisen. Dies ist im proximalen Abschnitt von besonderer Bedeutung, da dieser den größten Teil der Gesamtlänge des Ablöseschlauchs ausmacht, die Dehnbarkeit in Längsrichtung sollte daher möglichst gering sein, da sich ansonsten über die Gesamtlänge eine unerwünscht hohe Gesamtdehnung ergeben kann.

Von Vorteil ist entsprechend ein Ablöseschlauch mit einem distalen Abschnitt, der sich in das Implantat erstreckt, einem sich in proximaler Richtung anschließenden mittleren Abschnitt mit kleinem Außendurchmesser und einem sich in proximaler Richtung an den mittleren Abschnitt anschließenden proximalen Abschnitt mit großem Außendurchmesser. Der bei weitem längste Abschnitt, der hier als proximaler Abschnitt bezeichnet wird, weist einen großen Außendurchmesser auf, um ein Einführen und Zurückziehen des Ablöseschlauchs auch über größere Distanzen zu ermöglichen.

Typischerweise liegt die Länge des mittleren Abschnitts bei 50 bis 500 mm, insbesondere 80 bis 120 mm, besonders bevorzugt bei ca. 100 mm. Der distale Abschnitt kann bspw. eine Länge von 2 bis 10 mm haben. Die Gesamtlänge des Ablöseschlauchs kann 1.000 bis 2.000 mm, z. B. 1.800 mm betragen, entsprechend ist der proximale Abschnitt normalerweise der längste und weist eine Länge von 500 bis 1.900 mm auf.

Die Ausdrücke "großer Außendurchmesser" und "kleiner Außendurchmesser" sind erfindungsgemäß so zu verstehen, dass in Bereichen mit großem Außendurchmesser der Außendurchmesser größer ist als in Bereichen mit kleinem Außendurchmesser. Die genauen Maße können ebenso variieren wie das Verhältnis der Durchmesser, insbesondere in Abhängigkeit von den Verhältnissen im Blutgefäßsystem und dem konkreten Einsatzzweck. Ein typischer großer Außendurchmesser liegt aber in einem Bereich von 0,4 bis 0,8 mm, insbesondere 0,5 bis 0,7 mm, beispielsweise ca. 0,6 mm. Ein typischer kleiner Außendurchmesser liegt bei 0,3 bis 0,55, insbesondere 0,4 bis 0,5, beispielsweise ca. 0,45 mm.

An den proximalen Abschnitt des Ablöseschlauchs mit in der Regel großem Außendurchmesser kann sich noch ein proximales Ende anschließen, das wiederum einen verhältnismäßig kleinen Außendurchmesser aufweist. Hier wird der Ablöseschlauch sinnvollerweise auf den Einführdraht geklemmt, z. B. mit Hilfe eines Torquers, um einen Reibschluss zu erzeugen und eine unerwünschte gegenseitige Verschiebung von Einführdraht und Ablöseschlauch zu verhindern.

Um das Zurückziehen des Ablöseschlauchs zwecks Freisetzung des Implantats zu vereinfachen, kann am proximalen Ende des Ablöseschlauchs, unabhängig vom Außendurchmesser in diesem Bereich, eine Griffmöglichkeit vorgesehen sein. Diese kann in Form einer Verdickung oder als das proximale Ende des Ablöseschlauchs umgebende Hülse vorliegen. Wenn die Ablösung des Implantats erfolgen soll, wird der Torquer, der den Ablöseschlauch auf den Einführdraht klemmt, typischerweise gelöst und ggf. am Einführdraht neu angesetzt, um diesen besser handhabbar zu machen. Der Ablöseschlauch kann dann durch den Benutzer an der Griffmöglichkeit gefasst und in Richtung proximal zurückgezogen werden.

Die Passage des Implantats nebst Einführdraht und umgebendem Ablöseschlauch durch den Katheter kann dadurch erleichtert werden, dass eine Beschichtung des Ablöseschlauchs auf der Außenseite vorgesehen wird, die die Reibung zwischen Ablöseschlauch und Katheter verringert. Hierbei handelt es sich bevorzugt um eine hydrophile Beschichtung.

Beim Zurückziehen des Ablöseschlauchs ist es darüber hinaus sinnvoll, die Reibungskräfte zwischen Einführdraht und Ablöseschlauch möglichst gering zu halten. Hierfür kann, zumindest in Teilbereichen, eine reibungsmindernde Beschichtung auf der Außenseite des Einführdrahtes bzw. der Innenseite des Ablöseschlauchs zum Einsatz kommen. Bevorzugt ist die Verwendung von Polytetrafluorethylen (PTFE). Dies gilt insbesondere für Bereiche, in denen der Einführdraht angeschliffen ist, was typischerweise am proximalen Ende der Fall ist, um das Ergreifen mithilfe eines Torquers zu ermöglichen.

Neben dem Außendurchmesser kann auch die Wandstärke des Ablöseschlauchs variieren, d. h. in den Bereichen mit großem Außendurchmesser weist der Ablöseschlauch eine höhere Wandstärke auf als in den Bereichen mit kleinem Außendurchmesser. Die Verringerung der Wandstärke erhöht die Flexibilität und Biegsamkeit des Ablöseschlauchs zusätzlich, sodass er innerhalb des Mikrokatheters feinen Verzweigungen des Blutgefäßsystems besonders gut folgen kann.

Der Ablöseschlauch kann dadurch hergestellt werden, dass man von einem einheitlich aufgebauten Ablöseschlauch ausgeht, der zumindest über den größten Teil der Länge einen konstanten Außen- und Innendurchmesser, d. h. auch eine konstante Wandstärke aufweist. Von diesem Ablöseschlauch wird außen in den gewünschten Abschnitten Material abgetragen und somit der Außendurchmesser verringert. Da innen kein Material abgetragen wird, verringert sich in gleichem Maße die Wandstärke des Ablöseschlauchs. Man erhält somit einen Ablöseschlauch, der einstückig ist, wobei in Teilabschnitten, insbesondere im mittleren Abschnitt der Außendurchmesser sowie die Wandstärke durch Materialabtrag verringert wurden. In anderen Teilabschnitten, z. B. im proximalen und ggf. im distalen Abschnitt, wird normalerweise kein Material entfernt, d. h. der ursprüngliche Außendurchmesser bleibt hier erhalten.

Das Abtragen von Material kann mit Hilfe von grundsätzlich aus dem Stand der Technik bekannten Verfahren erfolgen, z. B. durch Abdrehen, Abschleifen oder Abschälen mit Hilfe mechanischer Werkzeuge oder auch mit Hilfe eines Lasers. Auch am proximalen Ende kann Material entfernt werden, um hier das Ergreifen durch einen Torquer zu ermöglichen.

Der Ablöseschlauch ist typischerweise aus einem Kunststoff gefertigt. Besonders bewährt haben sich Polyimide. Denkbar ist jedoch auch die Verwendung anderer Materialien, beispielsweise Polypropylen oder Polytetrafluorethylen (PTFE). Auch Kombinationen verschiedener Kunststoffe oder mehrschichtige, koextrudierte Polymere können verwendet werden. Darüber hinaus kann der Ablöseschlauch auch zusätzlich eine Bewehrung aufweisen, indem Fasern, beispielsweise Metallfasern in den Ablöseschlauch eingebettet werden. Denkbar ist z. B. ein Ablöseschlauch, der durch ein Gewebe oder Geflecht verstärkt wird.

Daneben kann der Ablöseschlauch auch aus Metall gefertigt sein, wobei der Ablöseschlauch dünnwandig ausgestaltet sein sollte, um keine zu große Biegesteifigkeit herbeizuführen. Insbesondere bieten sich als Metall Nickel-Titan-Legierungen wie Nitinol an.

Die genannten Materialien sind für den Ablöseschlauch verwendbar unabhängig davon, ob der Außendurchmesser des Ablöseschlauchs variiert.

Um die Biegesteifigkeit weiter zu verringern, kann der Ablöseschlauch Ausnehmungen oder Materialverdünnungen aufweisen, bspw. in Form von Schlitzen oder Öffnungen. Dies gilt unabhängig davon, aus welchem Material der Ablöseschlauch gefertigt ist, d. h. sowohl für Kunststoffe als auch für Metalle. Die Ausnehmungen/Materialverdünnungen können speziell in bestimmten Bereichen des Ablöseschlauchs vorgesehen werden, in denen eine geringe Biegesteifigkeit von besonderer Bedeutung ist, bspw. im distalen Bereich, oder auch über die gesamte Länge des Ablöseschlauchs angeordnet sein. Die Flexibiltät des Ablöseschlauchs wird auf diese Weise erhöht, ohne jedoch die Zugfestigkeit negativ zu beeinflussen.

Das Abtragen von Material kann in der Weise erfolgen, dass der Ablöseschlauch nach der Bearbeitung eine Vielzahl unterschiedlicher Außendurchmesser aufweist. Insbesondere kann der Übergang von Abschnitten mit großem Außendurchmesser zu Abschnitten mit kleinem Außendurchmesser und umgekehrt graduell erfolgen, beispielsweise über mehrere kleine Stufen, die jeweils geringfügig unterschiedliche Außendurchmesser aufweisen. Ebenso ist ein kontinuierlicher Übergang möglich, sodass sich der Außendurchmesser gleichmäßig verringert bzw. vergrößert. In diesem Fall ist der Übergang konisch. Im Längsschnitt betrachtet kann die Wandung des Ablöseschlauchs an den Stellen des Übergangs von großem zu kleinem Außendurchmesser eine Fase, einen schrägen oder auch einen runden oder bogenförmigen Verlauf aufweisen.

Alternativ kann der Ablöseschlauch auch mehrstückig aufgebaut sein. In diesem Fall werden Teilabschnitte des Ablöseschlauchs mit unterschiedlichen Außendurchmessern miteinander verbunden, in der Regel stoffschlüssig. Zweckmäßig ist eine Verbindung der Teilabschnitte mittels Klebung.

Beim Verbinden der Teilabschnitte mit unterschiedlichen Außendurchmessern sollten sich die Teilabschnitte überlappen, um eine sichere Verbindung, insbesondere eine ausreichende Haftfläche für die Klebung sicherzustellen. Ggf. kann der Innendurchmesser eines Teilabschnitts mit größerem Außendurchmesser erweitert werden, um das partielle Einführen eines Teilabschnitts mit kleinerem Durchmesser zu ermöglichen. Zusätzlich kann dafür gesorgt werden, dass die Übergänge zwischen den Teilabschnitten möglichst gleichmäßig verlaufen und sich der Außendurchmesser jeweils nicht sprunghaft, sondern sukzessive vergrößert bzw. verkleinert. Zu diesem Zweck können die Teilabschnitte angefast werden, möglich ist auch ein Materialabtrag in anderer Weise. Ebenso möglich ist es, eine gewisse zusätzliche Menge einer geeigneten Masse, bspw. des Klebstoffs aufzubringen, um auf diese Weise einen kontinuierlichen Übergang von großem zu kleinem Außendurchmesser zu erhalten.

Die Teilabschnitte können sich auch über längere Distanzen überlappen, beispielsweise kann eine Schicht des Ablöseschlauchs durchgehend über den größten Teil der Länge des Ablöseschlauchs verlaufen. Möglich ist eine Schicht, die am distalen Ende oder geringfügig proximal des distalen Endes des Ablöseschlauchs beginnt und bis zum proximalen Ende durchgehend verläuft und auf diese Weise einen weitgehend einheitlichen Innendurchmesser des Ablöseschlauchs gewährleistet. Ein einheitlicher Innendurchmesser ist fertigungstechnisch von Vorteil. In bestimmten Abschnitten, insbesondere im distalen und proximalen Abschnitt, wird auf die durchgehende Schicht des Ablöseschlauchs außen eine äußere Schicht des Ablöseschlauchs aufgebracht. Die inneren und äußeren Schichten werden miteinander verbunden, insbesondere verklebt. Dort, wo innere und äußere Schicht miteinander verbunden sind, erhält man somit einen Ablöseschlauch mit größerem Außendurchmesser und höherer Gesamtwandstärke, in den Abschnitten, wo keine äußere Schicht vorhanden ist, ist hingegen der Außendurchmesser und die Wandstärke kleiner. Es hat sich überraschend herausgestellt, dass ein mehrschichtiger Aufbau auch die Abschnitte des Ablöseschlauchs mit großem Außendurchmesser, insbesondere den proximalen Abschnitt, flexibler macht. Aufgrund der relativ hohen Wandstärke und damit verbunden großen Querschnittsfläche der Außenwand ist jedoch die Zugfestigkeit hoch. Im Vergleich zu einem einschichtigen Aufbau der Wandung des Ablöseschlauchs mit insgesamt gleicher Wandstärke ist somit die Flexibilität höher, die Zugfestigkeit jedoch vergleichbar.

Auch bei dieser Ausführungsform können selbstverständlich die Übergänge zwischen Abschnitten mit großem und kleinem Außendurchmesser kontinuierlich oder in Form mehrerer kleiner Stufen ausgebildet werden. Darüber hinaus kann der Ablöseschlauch neben innerer und äußerer Schicht weitere Schichten aufweisen, der Ablöseschlauch kann also grundsätzlich aus beliebig vielen Schichten aufgebaut sein.

Unabhängig von der genauen Ausgestaltung des Ablöseschlauchs ist der Abstand zwischen dem Einführdraht und der Innenwandung des Ablöseschlauchs insofern von Bedeutung, als bei zu großem Abstand, d. h. bei im Verhältnis zum Innendurchmesser des Ablöseschlauchs zu dünnem Einführdraht während des Vorschubs im Mikrokatheter ein Umbiegen oder Umknicken erfolgen kann, was im Extremfall den weiteren Vorschub unmöglich macht. Umgekehrt ist ein zu kleiner Abstand von Innenwandung des Ablöseschlauchs zum Einführdraht insofern problematisch, als bei Relativbewegungen hohe Reibungskräfte auftreten.

Es ist von Vorteil, wenn eine innere Schicht des Ablöseschlauchs zumindest weitgehend von distal nach proximal durchgehend verläuft. Hierunter wird verstanden, dass sich die innere Schicht über mindestens 70 %, vorzugsweise mindestens 80 % und besonders bevorzugt mindestens 90 % der Länge erstreckt. Als innere Schicht wird dabei nicht nur eine Schicht verstanden, die zunächst separat vorliegt und erst nachträglich mit einer äußeren Schicht verbunden wird, sondern auch der innere Teil eines einstückig ausgebildeten Ablöseschlauchs, wie er zuvor beschrieben wurde. Dies führt nicht nur zu einem einheitlichen Innendurchmesser, sondern darüber hinaus auch dazu, dass unerwünschte Längungen des Ablöseschlauchs beim proximalen Zurückziehen weitgehend vermieden werden. Auf der einen Seite sind die Abschnitte, in denen Biegsamkeit von besonderer Bedeutung ist, insbesondere der mittlere Abschnitt, besonders dünn und flexibel, sodass der Ablöseschlauch gut durch enge Blutgefäße geführt werden kann. Auf der anderen Seite sind weitere Abschnitte, insbesondere der proximale und ggf. der distale Abschnitt, hinreichend widerstandsfähig gegen eine unerwünschte Verlängerung des Ablöseschlauchs, wenn dieser nach proximal zurückgezogen wird. Dies gewährleistet eine sichere und problemlose Ablösung des Implantats.

Auch der Einführdraht kann in unterschiedlichen Abschnitten verschiedene Durchmesser aufweisen. Insbesondere kann der Durchmesser distal kleiner sein als im proximalen Abschnitt, da auch für den Einführdraht distal eine niedrige Biegesteifigkeit von Vorteil ist, damit er innerhalb des Mikrokatheters möglichst gut dem Verlauf des Blutgefäßes folgen kann. Auf der anderen Seite kann ein zu kleiner Durchmesser aber auch dazu führen, dass sich der Einführdraht beim Vorschieben verbiegt, wodurch der weitere Vorschub erschwert oder unmöglich gemacht wird. Es ist daher sinnvoll, den Einführdraht im distalen Abschnitt mit einem kleineren Durchmesser zu versehen, da der Einführdraht hier besonders darauf angewiesen ist, dem Verlauf des Blutgefäßes zu folgen, während im proximalen Abschnitt mehr der problemlose Vorschub im Vordergrund steht. Der Durchmesser kann auch über die Länge des Einführdrahtes mehrfach variieren, wobei an den Übergängen der Durchmesser vorzugsweise gleichmäßig zu- oder abnimmt. Die Übergänge sind somit bevorzugt konisch. Die Variation des Durchmessers des Einführdrahtes kann auch unabhängig von der Variation des Außendurchmessers des Ablöseschlauchs erfolgen.

Wenn auch grundsätzlich ein niedriger Durchmesser im distalen Abschnitt des Einführdrahtes von Vorteil ist, können einzelne Bereiche des Einführdrahtes auch im distalen Abschnitt wiederum einen größeren Durchmesser aufweisen. Dies gilt insbesondere für die Spitze des Einführdrahtes. Bei Einteilung des Einführdrahtes in eine proximale und eine distale Hälfte ist es aber sinnvoll, wenn der Durchmesser in der distalen Hälfte im Durchschnitt kleiner ist als in der proximalen Hälfte.

Die Bereiche des Einführdrahtes mit kleinem Durchmesser können mit einem Polymer, z. B. PTFE umhüllt sein. Auf diese Weise wird Spiel zwischen Einführdraht und Ablöseschlauch vermieden, wodurch eine unerwünschte Verformung des Einführdrahtes beim Vorschub unterbunden wird. Gleichwohl bleibt der Einführdraht in diesem Abschnitt ausreichend flexibel und biegsam, weil das Polymer den Einführdraht praktisch nicht versteift. Das Polymer kann auch in Form einer den Einführdraht ganz oder in Teilbereichen umgebenden spiralförmigen Wendel vorgesehen sein. Die spiralförmige Wendel kann auch aus einem anderen Material, insbesondere aus Metall bestehen.

Von Vorteil ist, wenn sich der Außendurchmesser des Ablöseschlauchs und der Durchmesser des Einführdrahtes in etwa synchron zueinander vergrößern oder verkleinern. Dies ist auch insofern sinnvoll, als in den gleichen Abschnitten von Ablöseschlauch einerseits und Einführdraht andererseits eine hohe Flexibilität wünschenswert ist. Darüber hinaus wird auf diese Weise gewährleistet, dass der Abstand zwischen Innenwandung des Ablöseschlauchs und Einführdraht relativ konstant bleibt. Der Durchmesser des Einführdrahtes kann distal durchaus erheblich abnehmen, sodass auch der Innendurchmesser des Ablöseschlauchs in den entsprechen Abschnitten klein sein kann; beispielsweise ist es möglich, dass der Ablöseschlauch im mittleren Abschnitt einen kleineren Innendurchmesser aufweist als der Durchmesser des Einführdrahts im proximalen Abschnitt.

Der Einführdraht kann sich auch durch das eigentliche, zur Ablösung bestimmte Implantat erstrecken. Insbesondere kann sich der Einführdraht nach distal auch über das distale Ende des Implantats hinaus erstrecken, wenn sich das Implantat im komprimierten Zustand befindet. Auf diese Weise wird bewirkt, dass auch nach Freisetzung des Implantats zunächst noch ein Objekt durch das Innere des Implantats verläuft, solange der Einführdraht nicht zurückgezogen wird. Dies ermöglicht die erneute Sondierung des Gefäßes bzw. Implantates, beispielsweise indem ein Katheter über den Einführdraht und die sich anschließende Einführdrahtspitze geführt wird. Der Katheter wird auf diese Weise durch das freigesetzte und expandierte Implantat bewegt. Die Einführdrahtspitze wird erst durch das endgültige Zurückziehen des Einführdrahtes entfernt.

Die Einführdrahtspitze kann ein rotationssymmetrisches Design haben. Der Querschnitt kann rund, oval, rechteckig oder auf grundsätzlich beliebige andere Art geformt sein. Sinnvoll ist es darüber hinaus, die Einführdrahtspitze visualisierbar zu machen, z. B. indem die Einführdrahtspitze selbst zumindest teilweise aus einem röntgensichtbaren Material gefertigt wird und/oder indem die Einführdrahtspitze an ihrem distalen Ende einen röntgendichten Marker aufweist. Hergestellt werden kann die Einführdrahtspitze aus Edelstahl, Nitinol, Platin, Platin/Iridium, Platin/Wolfram oder anderen Metallen.

Die Einführdrahtspitze und der eigentliche Einführdraht können einstückig gefertigt sein, d. h. es handelt es sich letztlich um einen durchgehenden Draht. Möglich ist es aber auch, Einführdrahtspitze und Einführdraht getrennt zu fertigen und erst nachträglich miteinander zu verbinden. Dabei können die vorteilhaften Eigenschaften unterschiedlicher Materialien miteinander kombiniert werden, bspw. kann der eigentliche Einführdraht aus Edelstahl mit guter Vorschiebbarkeit, die Einführdrahtspitze hingegen aus einer Nickel-Titan-Legierung wie Nitinol zur Erhöhung der Flexibilität bestehen. Dabei muss sich die Fertigung aus der Nickel-Titan-Legierung nicht auf die Einführdrahtspitze selbst beschränken, sondern kann insgesamt den distalen Abschnitt des Einführdrahtes betreffen. Der Einführdraht kann somit einen proximalen und einen distalen Abschnitt aufweisen, wobei beispielsweise der proximale Abschnitt aus Edelstahl, der distale Abschnitt aus einer Nickel-Titan-Legierung gefertigt ist. Ein distaler Abschnitt aus einer Nickel-Titan-Legierung weist auch den Vorteil auf, dass die Gefahr des Abknickens minimiert wird ("kink resistance"). Auf der anderen Seite ist für den proximalen Teil des Einführdrahtes die Verwendung eines steiferen Materials wie Edelstahl von Vorteil, weil dieses die Übertragung von Drehmomenten erlaubt, was für die Vorschiebbarkeit von Vorteil ist.

Der Einführdraht kann somit einen proximalen Abschnitt und einen distalen Abschnitt aufweisen, wobei der distale Abschnitt aus einer Nickel-Titan-Legierung, bevorzugt Nitinol, gefertigt ist, während der proximale Abschnitt aus einem steiferen Material gefertigt ist, d.h. aus einem Material mit höherem Elastizitätsmodul (Youngsches Modul). Insbesondere kann es sich bei dem Material für den proximalen Abschnitt um Edelstahl, aber auch um eine Co-Ni-Cr-Mo-Legierung wie MP35N, MP35NLT oder Elgiloy handeln.

Der Begriff Einführdraht ist weit zu verstehen und muss nicht in jedem Fall einen klassischen Draht bedeuten. Denkbar sind beispielsweise auch längliche Einführhilfen mit einem inneren Hohlraum. In diesem Fall entspricht der oben diskutierte Durchmesser des Einführdrahtes dem Außendurchmesser. Wichtig ist jedoch, dass sich der Einführdraht nach proximal so weit erstreckt, dass der behandelnde Arzt den Einführdraht ergreifen und bewegen kann.

Das zur Ablösung vorgesehene Implantat selbst weist vorzugsweise eine Wandung aus einzelnen, sich überschneidenden Filamenten auf, die ein röhrenförmiges Geflecht ausbilden. Das röhrenförmige Geflecht ist zumeist ein Rundgeflecht und hat einen vom proximalen oder distalen Ende aus betrachtet kreisförmigen Querschnitt. Grundsätzlich sind jedoch auch Abweichungen von der Kreisform möglich, beispielsweise ein ovaler Querschnitt.

Bei den Filamenten, die die Geflechtstruktur bilden, kann es sich um einzelne Drähte aus Metall handeln, möglich ist aber auch das Vorsehen von Litzen, d. h. mehreren Drähten geringen Durchmessers, die zusammen ein Filament bilden und vorzugsweise miteinander verdrillt sind.

Das Implantat wird im Folgenden anhand eines Flow Diverters beschrieben, der geeignet ist, den Blutfluss in einem Gefäß zu beeinflussen, dergestalt, dass arteriovenöse Fehlbildungen, soweit wie möglich vom Blutfluss abgeschottet werden. Bei den Fehlbildungen handelt es sich zumeist um Aneurysmen. Die erfindungsgemäße Vorrichtung ist hierauf jedoch nicht beschränkt und grundsätzlich auch für andere Implantate geeignet, die dafür vorgesehen sind, in Blutgefäße eingebracht und dort abgelöst zu werden, beispielsweise herkömmliche Stents, die eine Stützfunktion ausüben sollen.

Das Implantat kann auch dem Verschluss von Gefäßen dienen, die vom Blutkreislauf abgekoppelt werden sollen, weil sie beispielsweise Tumore versorgen. Das Implantat soll bei optimaler Auswahl des Verhältnisses von Implantatdurchmesser zu Gefäßdurchmesser in der Lage sein, sich an den jeweiligen Gefäßdurchmesser anzupassen. Im Bereich von Erweiterungen und Aussackungen soll es maximal seinen Nenndurchmesser annehmen, d. h. den Durchmesser, den das Implantat ohne Ausübung eines äußeren Zwangs einnimmt.

Als Material für das Implantat kommen insbesondere Materialien mit hoher Rückstellkraft bzw. Federwirkung in Frage. Dies sind insbesondere Materialien mit superelastischen oder Formgedächtniseigenschaften, beispielsweise Nitinol. Dabei können für die einzelnen Filamente auch Drähte mit unterschiedlichem Durchmesser verwendet werden. Die Vor- und Nachteile von Drähten mit unterschiedlichem Querschnitt können auf diese Weise kombiniert bzw. kompensiert werden. Der Querschnitt der Drähte ist in den meisten Fällen rund, möglich sind aber auch Drähte mit ovalem oder eckigem Querschnitt oder Kombinationen hieraus.

Wichtig ist in jedem Fall, dass das Implantat einerseits in der Lage ist, eine komprimierte Form zwecks Durchführung durch den Mikrokatheter einzunehmen, sich andererseits aber bei Befreiung vom äußeren Zwang des Mikrokatheters automatisch aufzuweiten und sich am Implantationsort an die Gefäßinnenwandung anzulegen. Möglich ist auch die Fertigung des Implantats aus Verbundmaterialien, beispielsweise aus mit Platin ummantelten Nickel-Titan-Drähten oder mit Nickel-Titan ummantelten Platindrähten. Auf diese Weise werden die Formgedächtniseigenschaften der Nickel-Titan-Legierung (Nitinol) mit der Röntgensichtbarkeit des Platins kombiniert.

Der Durchmesser des Implantats im expandierten Zustand liegt typischerweise zwischen 2,5 und 5,0 mm. Die Länge beträgt beispielsweise 20 bis 40 mm

Der Einführdraht kann aus Edelstahl oder auch aus einem Formgedächtniswerkstoff, insbesondere einer Nickel-Titan-Legierung wie Nitinol gefertigt sein. Bei Einführdrähten, deren Durchmesser variiert, ist es sowohl möglich, den Einführdraht aus einem einzelnen Draht zu schleifen, d. h. in den Bereichen kleineren Durchmessers Material abzutragen. Möglich ist aber auch die Zusammenfügung von mehreren einzelnen Drähten zu einem Einführdraht an den Stellen, an denen sich der Durchmesser des Einführdrahtes ändert. Dabei können unterschiedliche Materialien zum Einsatz kommen. Insbesondere ist es möglich, einen Einführdraht aus Edelstahl mit einer Spitze aus einer Nickel-Titan-Legierung am distalen Ende zu versehen bzw. allgemein weiter distal liegende Bereiche des Einführdrahtes aus einer Nickel-Titan-Legierung, weiter proximal liegende Bereiche aus einem Material mit höherem Elastizitätsmodul wie Edelstahl zu fertigen.

Wenn das Implantat als Flow Diverter dient, muss es nicht unbedingt, wie es bei üblichen Stents der Fall ist, eine Stützfunktion ausüben. Das Implantat dient vielmehr in erster Linie zur Kanalisierung des Blutflusses im Bereich der Fehlbildungen im Sinne einer Art innerer Manschette. Es soll beispielsweise auch in einem Aneurysma platzierte Okklusionsmittel daran hindern, in die Gefäßbahn ausgeschwemmt zu werden. Darüber hinaus kann der Ein- und/oder Ausfluss von Blut in ein Aneurysma verhindert werden.

Die Implantate werden typischerweise als Geflecht aus einer Mehrzahl von Filamenten gefertigt, wobei das Geflecht im Prinzip einen Endlosschlauch bildet. Die jeweils benötigte Implantatlänge kann dann aus diesem Endlosschlauch abgelängt werden. Die einzelnen Filamente sind dazu spiral- oder helixförmig aufgewickelt, wobei die einzelnen Filamente als Flechtwerk, d. h. einander kreuzend untereinander und übereinander eingebracht werden. In der Regel sind die einzelnen Filamente dabei in zwei einander in einem konstanten Winkel kreuzenden Richtungen gewickelt, die sich beispielsweise in einem Winkel von 90° schneiden. Bevorzugt sind - im spannungsfreien Normalzustand - Winkel von mehr als 90°, insbesondere von 90 bis 160°, wobei die zu den axialen Enden des Implantats hin offenen Winkel gemeint sind. Eine solche steile Wicklung der Einzelfilamente kann, wenn sie hinreichend dicht ist, zu einem Geflecht mit hoher Oberflächendichte bzw. Oberflächenabdeckung führen, das bei axialer Streckung zu erheblich geringeren Durchmessern auseinandergezogen werden kann. Bei Wegfall der Streckkräfte und hinreichender Rückstellkraft des Filamentmaterials nähert sich das Geflecht dem Nenndurchmesser, d. h. dem ursprünglich spannungsfreien Zustand, wieder an und dehnt sich aus, was zu einer engen Anschmiegung an die Gefäßwand am Implantationsort und zu einer Verdichtung der Maschenstruktur an der Wandung führt. Dies gilt insbesondere auch im Bereich von Gefäßerweiterungen. Die Oberflächenabdeckung ist somit in den Bereichen einer Gefäßerweiterung, beispielsweise eines Aneurysmas höher als in angrenzenden Bereichen des Gefäßes. Zusätzlich oder alternativ kann die Oberflächenabdeckung des Geflechts auch durch die angewandte Flechttechnik variiert werden. Beispielsweise kann das Implantat im mittleren Bereich, in dem typischerweise das Aneurysma abgedeckt wird, dichter geflochten sein als in den Endbereichen, sodass eine weitgehende Abdeckung des Aneurysmahalses gewährleistet ist. Auf der anderen Seite wird durch eine geringere Oberflächendichte in den Endbereichen eine hinreichende Flexibilität gewährleistet. Gefäßabzweigungen (Bifurkationen) können bei den Implantaten beispielsweise durch Bereiche einer geringeren Maschendichte berücksichtigt werden. Die Stärke der Filamente beträgt typischerweise 0,01 bis 0,2 mm, insbesondere 0,02 bis 0,05 mm. Jedes Einzelfilament kann aus einem einzelnen Draht oder auch aus einer Litze von mehreren zusammengefassten und vorzugsweise miteinander verdrillten Einzeldrähten bestehen. Die Einzeldrähte können den gleichen Durchmesser sowie auch unterschiedliche Durchmesser aufweisen. Auch können die Drähte aus unterschiedlichen Materialien (Nitinol, Cobalt-Chrom-Legierungen, Platin-Legierungen) bestehen. Drähte aus einem röntgendichten Material beispielsweise sorgen für die Röntgensichtbarkeit des Implantats.

In dem Geflecht können an den Implantatenden herausstehende Filamentenden zumindest paarweise zusammengeführt und miteinander dauerhaft verbunden werden. Dies kann beispielsweise durch Verschweißen erfolgen, aber auch durch mechanisches Verklammern, Verdrillen, Verlöten oder Verkleben. Eine Verbindung der Filamentenden kann auch mittels einer aufgesetzten Hülse erfolgen. Diese Hülse kann mit den Filamentenden eine stoffschlüssige Verbindung eingehen, beispielsweise verschweißt oder auch vercrimpt sein. Eine Alternative besteht darin, dass die Hülse so dimensioniert ist, dass an den Filamentenden befindliche Verdickungen daran gehindert sind, durch die Hülse hindurchzurutschen. Die Hülse ist somit relativ zu den Filamenten in axialer Richtung verschiebbar, sie kann aber nicht vollständig abgezogen werden. Des Weiteren können die Hülsen in axialer Richtung gegeneinander versetzbar sein. Auf diese Weise wird erreicht, dass die Hülsen beim Komprimieren des Implantats nicht unmittelbar übereinander zu liegen kommen, sodass das Implantat insgesamt einen geringeren Durchmesser aufweist.

Von Bedeutung ist das Zusammenführen und Verbinden der Filamentenden insbesondere am proximalen Ende des Implantats; es hat sich herausgestellt, dass am distalen Ende des Implantats auch freie Filamentenden unproblematisch sind. Gleichwohl ist es selbstverständlich möglich, auch am distalen Ende des Implantats die Filamentenden zusammenzuführen und miteinander zu verbinden.

Möglich ist auch eine Zusammenführung der Filamente zu ersten Geflechtenden, die wiederum zu zweiten Geflechtenden verbunden sind, wie in der DE 10 2009 006 180 A1 beschrieben.

Dabei oder zusätzlich können die verbundenen Filamentenden atraumatisch umgeformt werden. Insbesondere können die Filamentenden distal und/oder proximal eine atraumatische Verdickung aufweisen, die z. B. ungefähr kugelförmig ist. Die Verdickung kann durch Laserschweißen, Hartlöten, Verkleben, Aufcrimpen o. ä. aus dem Filamentende ausgeformt oder am Filamentende angebracht werden.

Die Platzierung der erfindungsgemäßen Implantate erfolgt in der Praxis unter Röntgenkontrolle. Aus diesem Grund sollte das Implantat und ggf. auch der Einführdraht ein röntgendichtes Markermaterial aufweisen, sofern es nicht aus einem röntgendichten Material selbst gefertigt ist. Solche röntgendichten Materialien sind insbesondere Tantal, Gold, Wolfram und Platinmetalle, insbesondere Platinlegierungen wie Platin-Iridium oder Platin-Wolfram. Diese Marker können beispielsweise als Markerelemente in bekannter Weise an die Filamentenden angeheftet werden, aber auch als Markerfilamente in die Geflechtstruktur des Implantates eingeflochten werden. Die Ummantelung einzelner Filamente mit einer Helix oder Draht aus einem röntgendichten Material wie Platin ist ebenfalls möglich. Die Helix bzw. der Draht kann mit den Filamenten verschweißt, verklebt o. ä. sein. Eine weitere Möglichkeit besteht in der Beschichtung oder Füllung der Filamente mit einem röntgendichten Material.

Ebenfalls möglich sind röntgendichte Markierungen in Form von Hülsen, die die zusammengeführten Filamente umschließen. Diese Hülsen können mit den Filamentenden verschweißt oder auch vercrimpt sein. Die röntgendichten Hülsen können mit den oben erwähnten Hülsen zum Zusammenhalten der Filamentenden identisch sein und somit eine Doppelfunktion erfüllen. Möglich ist darüber hinaus das Versehen eines distalen Abschnitts des Einführdrahtes mit einer Wendel aus röntgendichtem Material, bspw. einer Pt-Wendel.

Denkbar ist auch die Einbringung röntgensichtbarer Substanzen in den Ablöseschlauch. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit des Ablöseschlauchs ist bei der Einbringung und Lokalisierung des Implantats hilfreich und kann zusätzlich oder anstelle von Markerelementen Verwendung finden.

Wie vorstehend beschrieben, kommt es für den Verschluss von Aneurysmen bei der spannungsfreien Anordnung der Einzelfilamente im Geflecht darauf an, die Implantatoberfläche möglichst dicht zu gestalten. Da die Flexibilität des Geflechts erhalten bleiben muss, ist eine 100 %ige Oberflächenabdeckung durch die Filamente allerdings allenfalls annähernd möglich. Je nach Anwendung können sich aber auch geringere Oberflächenabdeckungen ergeben bzw. haben sich auch geringere Oberflächenabdeckungen als ausreichend herausgestellt. Bevorzugt ist eine Oberflächenabdeckung im Bereich von 30 bis 80 %, vorzugsweise von 35 bis 70 %.

Zur Verbesserung der Oberflächenabdeckung kann das Geflecht mit einer Folie ummantelt werden, beispielsweise aus Teflon, Silikon oder einem anderen körperverträglichen Kunststoff. Zur Erhöhung der Flexibilität und Dehnbarkeit kann eine solche Kunststofffolie geschlitzt sein, wobei die Schlitzanordnung gestaffelt ist und die Längsrichtung der Schlitze entlang der Umfangslinie des Implantates verläuft. Eine solche Folie kann beispielsweise durch Eintauchen des Implantates in ein entsprechendes flüssiges Folienmaterial (Dispersion oder Lösung) und anschließende Einfügung der Schlitze, beispielsweise mit einem Laser erzielt werden. Durch Eintauchen kann beispielsweise auch eine vollständige oder teilweise Füllung der Maschen erreicht werden.

Alternativ ist es möglich, die einzelnen Filamente des Implantats durch Eintauchen in eine Kunststoffdispersion oder Lösung mit einem solchen Kunststoff zu ummanteln und dadurch den Filamentquerschnitt zu erhöhen. In diesem Fall bleiben offene Maschen, jedoch wird die Maschengröße deutlich vermindert.

Das Implantat kann auf an und für sich bekannte Weise beschichtet sein. Als Beschichtungsmaterialien kommen insbesondere solche in Frage, wie sie für Stents beschrieben sind, etwa mit antiproliferativen, entzündungshemmenden, antithrombogenen, das Einwachsen fördernden und/oder die Anlagerung hindernden, hämokompatiblen Eigenschaften. Bevorzugt ist eine Beschichtung, die das Einwachsen des Implantats und die Neointimabildung fördert. Es kann sinnvoll sein, das Implantat außen derart zu beschichten und innen mit einem Mittel, das die Anhaftung mindert, etwa Heparin oder einem Derivat, ASS oder dazu geeigneten Oligosacchariden und Chitinderivaten. Hier geeignet sind ferner Schichten aus Nano-Partikeln, etwa ultradünnen Schichten aus polymerem SiO₂, die die Anhaftung mindern.

Die Erfindung wird anhand der nachfolgenden Darstellungen beispielhaft näher erläutert. Es zeigen:
- Figur 1:: eine erfindungsgemäße Vorrichtung im Längsschnitt;
- Figuren 2 - 7:: verschiedene Varianten eines Ablöseschlauchs gemäß der Erfindung und
- Figur 8:: eine Variante der Erfindung, bei der ein Ablösedraht verwendet wird.

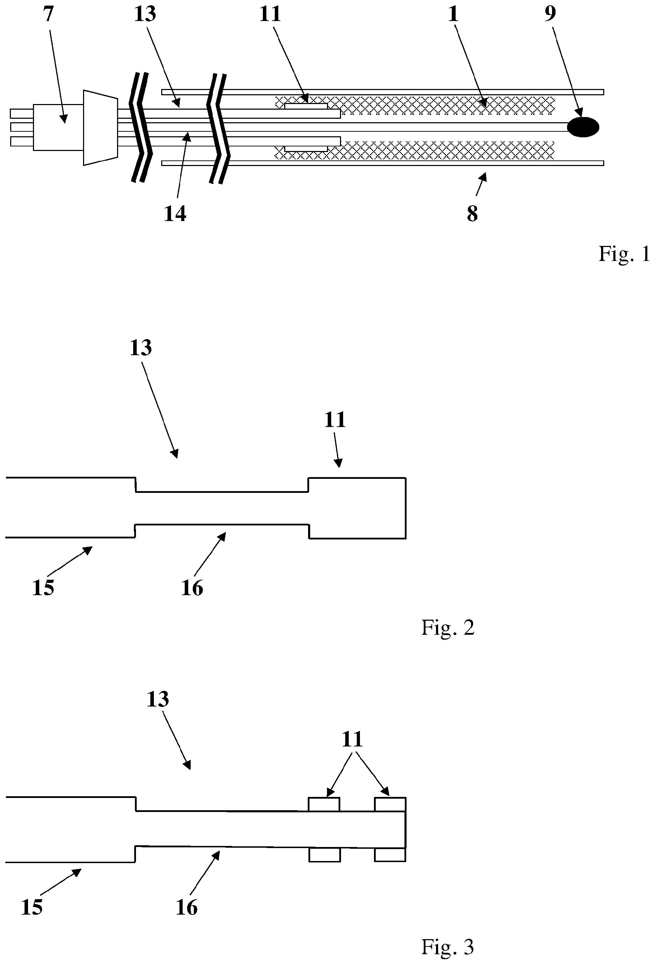

In den Figuren 1 - 7 ist die Erfindung dargestellt. Der Ablöseschlauch 13 weist an seinem distalen Ende ein oder mehrere Pads 11 auf, die aus einem elastischen Material gefertigt sind und einen ausreichend hohen Reibschluss zwischen Pad 11 und Implantat 1 erzeugen, sodass ein Vorschieben und Zurückziehen des Implantates 1 durch Bewegung des Ablöseschlauchs 13 möglich ist. Das Implantat 1 befindet sich in der hier gewählten Darstellung innerhalb des Mikrokatheters 8, d. h. in seiner komprimierten Form. Der Einführdraht 14 erstreckt sich hier durch das gesamte Implantat, sodass die Einführdrahtspitze 9 distal des distalen Endes des Implantates liegt, eine Einführdrahtspitze 9 ist aber nicht obligatorisch. Bei der Darstellung gemäß den Figuren 1 - 7 liegt proximal links und distal rechts. Am proximalen Ende der Vorrichtung wird der Einführdraht 14 sowie der Ablöseschlauch 13 mit Hilfe eines Torquers 7 gehalten.

Sobald das Implantat 1 am Zielort angekommen ist, kann es aus dem Mikrokatheter 8 nach distal hinausgeschoben bzw. der Mikrokatheter 8 nach proximal zurückgezogen werden, sodass sich das Implantat 1 radial aufweitet und sich an die Gefäßinnenwand anpasst. Das Ablöseprinzip basiert somit darauf, dass eine Ablösung durch eine Aufweitung des Implantates 1 in radialer Richtung erfolgt, während in axialer Richtung ausreichend hohe Reibungskräfte erzeugt werden, die eine Freisetzung des Implantates 1 in axialer Richtung ausschließen.

In den Figuren 2 - 7 sind verschiedene Varianten von Ablöseschläuchen 13 dargestellt. In der in Figur 2 dargestellten Ausführungsform weist ein proximaler Abschnitt 15 des Ablöseschlauches 13 einen höheren, ein weiter distal liegender mittlerer Abschnitt 16 hingegen einen niedrigeren Querschnitt auf. Dies bewirkt, dass auf der einen Seite der mittlere Abschnitt 16 des Ablöseschlauches 13 recht flexibel ist und sich beim Transport durch englumige Blutgefäße problemlos vorschieben lässt, auf der anderen Seite lässt sich der Ablöseschlauch 13 auch problemlos in Richtung proximal zurückziehen, da der proximale Abschnitt 15 einen höheren Querschnitt aufweist und somit die Längsdehnbarkeit des Ablöseschlauches 13 begrenzt. Gemäß Figur 2 ist das Pad 11 aus dem Schlauchmaterial selbst gefertigt d. h. es muss kein Pad 11 zusätzlich auf dem Ablöseschlauch 13 angebracht werden.

Figur 3 stellt eine ähnliche Ausführungsform dar, bei der ebenfalls der mittlere Abschnitt 16 einen niedrigeren Querschnitt aufweist als der proximale Abschnitt 15 des Ablöseschlauches 13. Im Gegensatz zur in Figur 2 dargestellten Ausführungsform sind hier jedoch zwei Pads 11 separat angebracht, die den Ablöseschlauch 13 ringförmig umgeben.

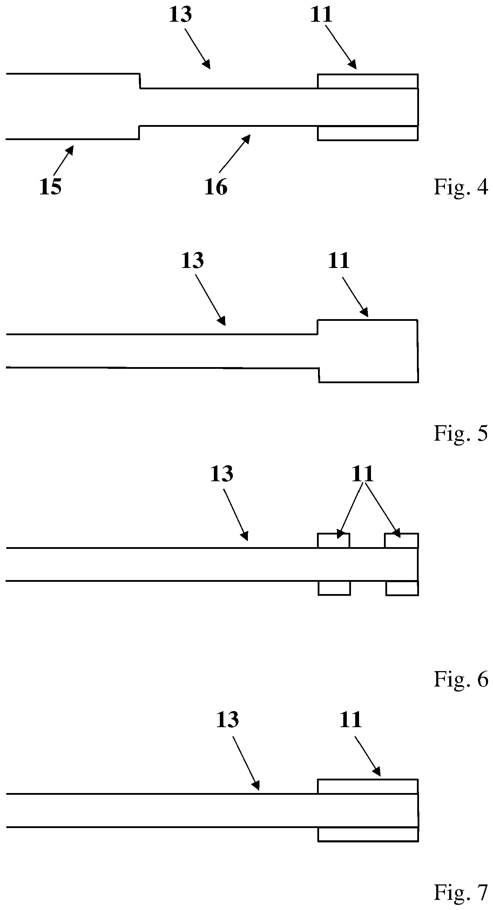

In Figur 4 ist eine weitere Variante dargestellt, bei der ebenfalls der proximale Abschnitt 15 einen höheren Querschnitt aufweist als der weiter distal liegende mittlere Abschnitt 16 des Ablöseschlauches 13, hier wurde jedoch nur ein den Ablöseschlauch 13 ringförmig umgebendes Pad 11 aufgebracht, welches länger ausgebildet ist als die einzelnen Pads gemäß Figur 3.

Die Figuren 5, 6 und 7 entsprechen den Figuren 2, 3 und 4, wobei aber der Ablöseschlauch 13 keinen Absatz aufweist und, vom distalen Ende abgesehen, einen einheitlichen Querschnitt hat.

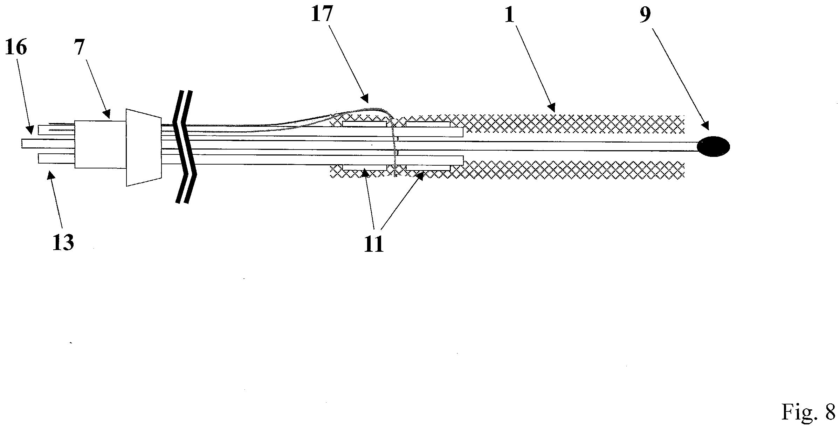

In Figur 8 wird eine Variante dargestellt, bei der ein Reibschluss zwischen Implantat 1 und Ablöseschlauch 13 erzeugt wird, indem ein Ablösedraht 17 um das Implantat 1 geschlungen wird. Der Ablösedraht 17 schnürt dabei das Implantat 1 zwischen zwei Pads 11 so weit ein, dass eine Fixierung des Implantates 1 auf dem Ablöseschlauch 13 gegeben ist. Auch ohne Mikrokatheter bleibt somit das Implantat 1 auf dem Ablöseschlauch 13 festgelegt, bis durch Anlegen einer elektrischen Spannung eine elektrolytische Korrosion des Ablösedrahtes 17 an einer hierfür vorgesehenen Ablösestelle erfolgt und der Ablösedraht sich vom Implantat 1 löst. Anschließend kann sich das Implantat 1 aufweiten und der Mikrokatheter, der Ablöseschlauch 13, der Einführdraht 14 sowie die verbleibenden Enden des Ablösedrahtes 17 werden zurückgezogen. Um das Anlegen einer elektrischen Spannungsquelle an den Ablösedraht 17 zu ermöglichen, verlaufen die beiden Enden des Ablösedrahtes 17 in Richtung proximal.

## Patentansprüche

1. Vorrichtung zur Einbringung eines Implantates (1) in Blutgefäße oder Hohlorgane des menschlichen oder tierischen Körpers, umfassend ein Implantat (1) und einen Ablöseschlauch (13), wobei das Implantat (1) in der Weise verformbar ist, dass es in einem Mikrokatheter (8) eine Form mit vermindertem Durchmesser annimmt und am Ort der Implantation nach Wegfall des äußeren Zwangs durch den Mikrokatheter (8) unter Anpassung an den Durchmesser des Blutgefäßes oder Hohlorganes expandiert, und der Ablöseschlauch (13) ein in Längsrichtung der Vorrichtung verlaufendes Lumen aufweist, durch welches ein Einführdraht (14) längsverschieblich führbar ist, **dadurch gekennzeichnet, dass** der Ablöseschlauch (13) in das proximale Ende des Implantates (1) hineinragt, wobei eine elastische Kontaktfläche zwischen der Innenseite des Implantates (1) und der Außenseite des Ablöseschlauchs (13) vorliegt, sodass ein Reibschluss zwischen Implantat (1) und Ablöseschlauch (13) erzeugt wird, der eine Längsverschieblichkeit des Implantates (1) innerhalb des Mikrokatheters (8) durch Längsbewegung des Ablöseschlauchs (13) nach distal oder proximal herbeiführt.

2. Vorrichtung nach Anspruch 1, **dadurch gekennzeichnet, dass** der Ablöseschlauch (13) am proximalen Ende auf den Einführdraht (14) klemmbar ist.

3. Vorrichtung nach Anspruch 1 oder 2, **dadurch gekennzeichnet, dass** die elastische Kontaktfläche zwischen der Innenseite des Implantates (1) und der Außenseite des Ablöseschlauchs (13) durch eine elastische Zwischenlage (11) hergestellt ist.

4. Vorrichtung nach Anspruch 3, **dadurch gekennzeichnet, dass** die Zwischenlage (11) ringförmig um den Ablöseschlauch (13) verläuft.

5. Vorrichtung nach Anspruch 3 oder 4, **dadurch gekennzeichnet, dass** die Zwischenlage (11) aus demselben Material besteht wie der Ablöseschlauch (13), insbesondere aus einem Polyimid.

6. Vorrichtung nach Anspruch 3 oder 4, **dadurch gekennzeichnet, dass** die Zwischenlage (11) aus einem Elastomer besteht.

7. Vorrichtung nach Anspruch 6, **dadurch gekennzeichnet, dass** das Elastomer Gummi, Kautschuk oder ein Silikon ist.

8. Vorrichtung nach einem der Ansprüche 1 bis 7, **dadurch gekennzeichnet, dass** ein Ablösedraht (17) in dem Bereich, in dem der Ablöseschlauch (13) in das proximale Ende des Implantats (1) hineinragt und in dem eine elastische Kontaktfläche zwischen der Innenseite des Implantates (1) und der Außenseite des Ablöseschlauchs (13) vorliegt, um das Implantat (1) geschlungen ist, sodass der Ablösedraht (17) eine Verstärkung der Reibungskräfte zwischen Implantat (1) und Ablöseschlauch (13) erzeugt, wobei der Ablösedraht (17) an zumindest einer Ablösestelle elektrolytisch korrodierbar oder thermisch durchtrennbar ausgebildet ist.

9. Vorrichtung nach einem der Ansprüche 1 bis 8, **dadurch gekennzeichnet, dass** der Außendurchmesser des Ablöseschlauchs (13) zwischen proximalem und distalem Ende variiert.

10. Vorrichtung nach einem der Ansprüche 1 bis 9, **dadurch gekennzeichnet, dass** das Implantat (1) ein Geflecht aus einer Vielzahl an Geflechtdrähten (6) ist.

## Claims

1. A device for introducing an implant (1) into blood vessels or hollow organs of the human or animal body, comprising an implant (1) and a release tube (13), wherein the implant (1) is deformable so that in a microcatheter (8) it adopts a shape with reduced diameter and at the implantation site it expands once the external constraint of the microcatheter (8) disappears, adapting to the diameter of the blood vessel or hollow organ, and the release tube (13) has a lumen running in the longitudinal direction of the device, through which an insertion wire (14) can be guided in a longitudinally displaceable manner, **characterised in that** the release tube (13) protrudes into the proximal end of the implant (1), wherein an elastic contact surface is present between the inside of the implant (1) and the outside of the release tube (13), so that a frictional lock is generated between the implant (1) and the release tube (13), bringing about a longitudinal displaceability of the implant (1) inside the microcatheter (8) by longitudinal movement of the release tube (13) distally or proximally.

2. The device according to claim 1, **characterised in that** the release tube (13) can be clamped at the proximal end on the insertion wire (14).

3. The device according to claim 1 or 2, **characterised in that** the elastic contact surface between the inside of the implant (1) and the outside of the release tube (13) is produced by an elastic intermediate layer (11).

4. The device according to claim 3, **characterised in that** the intermediate layer (11) runs in a ring around the release tube (13).

5. The device according to claim 3 or 4, **characterised in that** the intermediate layer (11) consists of the same material as the release tube (13), in particular a polyimide.

6. The device according to claim 3 or 4, **characterised in that** the intermediate layer (11) consists of an elastomer.

7. The device according to claim 6, **characterised in that** the elastomer is a vulcanised rubber, an unvulcanised rubber or a silicone.

8. The device according to one of claims 1 to 7, **characterised in that** a release wire (17) is wound around the implant (1) in the region in which the release tube (13) protrudes into the proximal end of the implant (1) and in which an elastic contact surface is present between the inside of the implant (1) and the outside of the release tube (13), so that the release wire (17) produces a strengthening of the frictional forces between the implant (1) and the release tube (13), wherein the release wire (17) is electrolytically corrodible or thermally severable at least at one release site.

9. The device according to one of claims 1 to 8, **characterised in that** the outside diameter of the release tube (13) varies between the proximal and distal end.

10. The device according to one of claims 1 to 9, **characterised in that** the implant (1) is a braid made from a plurality of braided wires (6).

## Revendications

1. Dispositif d'insertion d'un implant (1) dans des vaisseaux sanguins ou organes creux du corps humain ou animal, comprenant un implant (1) et un tuyau de séparation (13), l'implant (1) pouvant être déformé de manière à prendre une forme avec un diamètre réduit dans un microcathéter (8) et à se dilater à l'endroit de l'implantation après la suppression de la contrainte extérieure exercée par le microcathéter (8) en s'adaptant au diamètre du vaisseau sanguin ou de l'organe creux, et le tuyau de séparation (13) présentant une lumière s'étendant dans la direction longitudinale du dispositif, un fil d'introduction (14) pouvant être guidé de manière déplaçable longitudinalement dans ladite lumière, **caractérisé en ce que** le tuyau de séparation (13) dépasse dans l'extrémité proximale de l'implant (1), une surface de contact élastique étant présente entre le côté intérieur de l'implant (1) et le côté extérieur du tuyau de séparation (13), de telle sorte qu'une liaison par friction est générée entre l'implant (1) et le tuyau de séparation (13), laquelle provoque une déplaçabilité longitudinale de l'implant (1) à l'intérieur du microcathéter (8) par un mouvement longitudinal du tuyau de séparation (13) dans la direction distale ou proximale.

2. Dispositif selon la revendication 1, **caractérisé en ce que** le tuyau de séparation (13) peut être serré sur le fil d'introduction (14) à l'extrémité proximale.

3. Dispositif selon la revendication 1 ou 2, **caractérisé en ce que** la surface de contact élastique entre le côté intérieur de l'implant (1) et le côté extérieur du tuyau de séparation (13) est réalisée par une couche intermédiaire (11) élastique.

4. Dispositif selon la revendication 3, **caractérisé en ce que** la couche intermédiaire (11) s'étend en forme d'anneau autour du tuyau de séparation (13).

5. Dispositif selon la revendication 3 ou 4, **caractérisé en ce que** la couche intermédiaire (11) est constituée du même matériau que le tuyau de séparation (13), en particulier un polyimide.

6. Dispositif selon la revendication 3 ou 4, **caractérisé en ce que** la couche intermédiaire (11) est constituée d'un élastomère.

7. Dispositif selon la revendication 6, **caractérisé en ce que** l'élastomère est de la gomme, du caoutchouc ou un silicone.

8. Dispositif selon l'une des revendications 1 à 7, **caractérisé en ce qu'**un fil de séparation (17) est enroulé autour de l'implant (1) dans la zone dans laquelle le tuyau de séparation (13) dépasse dans l'extrémité proximale de l'implant (1) et dans laquelle une surface de contact élastique est présente entre le côté intérieur de l'implant (1) et le côté extérieur du tuyau de séparation (13), de telle sorte que le fil de séparation (17) génère un renforcement des forces de frottement entre l'implant (1) et le tuyau de séparation (13), le fil de séparation (17) étant réalisé corrodable électrolytiquement ou séparable thermiquement en au moins un point de séparation.

9. Dispositif selon l'une des revendications 1 à 8, **caractérisé en ce que** le diamètre extérieur du tuyau de séparation (13) varie entre l'extrémité proximale et distale.

10. Dispositif selon l'une des revendications 1 à 9, **caractérisé en ce que** l'implant (1) est un tressage constitué d'une pluralité de fils de tressage (6).
